# EUROPEAN PATENT SPECIFICATION

(11) **EP 1 405 647 B1**
(45) Date of publication and mention of the grant of the patent: **05.04.2006**
(21) Application number: 02079105.9
(22) Date of filing: 03.10.2002
(51) Int. Cl.: A61L 27/10, C03C 3/097, C03C 4/00

(54) **Bioactive glass composition**
Bioaktive Glaszusammensetzung
Composition de verre bioactif

(43) Date of publication of application: 07.04.2004
(73) Proprietor: Vivoxid Oy, 20520 Turku (FI)
(72) Inventor: Ylänen, Heimo, 20810 Turku (FI); Yli-Urpo, Antti, 20660 Littoinen (FI); Hupa, Mikko, 20720 Turku (FI)
(74) Representative: Heikkilä, Hannes Antero

(56) References cited:
- WO-A-01/66479
- WO-A-96/21628
- ITALA ARI ET AL: "Creation of microrough surface on sintered bioactive glass microspheres." JOURNAL OF BIOMEDICAL MATERIALS RESEARCH, vol. 56, no. 2, August 2001 (2001-08), pages 282-288, XP002235416 ISSN: 0021-9304

## Description

### FIELD OF THE INVENTION

This invention relates to a bioactive glass composition comprising SiO₂, Na₂O, CaO, K₂O, MgO, P₂O₅ and B₂O₃. The invention further relates to the use of said composition and devices manufactured from it. The invention still relates to a method for manufacturing a bioactive glass composition according to the present invention.

### BACKGROUND OF THE INVENTION

In this application, by bioactive glass is meant a material that has been designed to induce specific biological activity in body tissue. The term biodegradable in this context means that it is degradable upon prolonged implantation when inserted into the mammal body. By biomaterial a non-viable material used in a medical device is meant, a material that is intended to interact with biological systems.

Glasses have been studied extensively for applications in medical and dental surgery and implants. A medical device can be implanted into any human or animal tissue. This allows local application so that targeting of the biologically active agent release site is possible. Since only a non-crystallized glass composition shows the best bioactivity and since bioactive glass compositions are in the area near the phase separation, it is very difficult to make glass compositions that do not crystallize during repeated heat treatment, i.e. that remain bioactive.

Bioactive glasses develop reactive layers on their surfaces resulting in bonding between the device and the host tissue. Unlike most other bioactive materials; the rate of chemical reactions of bioactive glasses can be easily controlled by changing the chemical composition of the glass. Therefore, bioactive glasses are interesting in particular in clinical applications and have indeed been used for example to replace damaged parts of facial injuries, replacement of the small bones (ossicles) in the middle ear and in surgery to fill defects in bone.

Unfortunately, the traditionally known bioactive glass compositions do not support repeated heat-treatments, since reheating results in a decrease of the bioactivity. This causes great problems in the manufacturing of devices from these compositions, since they can only be shaped by molding them into the final shape already in the production step of the glass or by crushing the previously formed glass particles. The molding process only allows the production of rigid non-porous devices.

An improved bioactive glass composition with respect to the heat-treating properties has been presented by Brink et al. in WO 96/21628. This document discloses a bioactive glass of the following composition:
SiO₂ in an amount of 53 - 60 wt-%,
Na₂O in an amount of 0 - 34 wt-%,
K₂O in an amount of 1 - 20 wt-%,
MgO in an amount of 0 - 5 wt-%,
CaO in an amount of 5 - 25 wt-%,
B₂O₃ in an amount of 0 - 4 wt-%,
P₂O₅ in an amount of 0,5 - 6 wt-%,
provided that
Na₂O + K₂O = 16 - 35 wt-%
K₂O + MgO = 5 - 20 wt-%, and
MgO + CaO = 10 - 25 wt-%.

The heat-treating properties of these glasses are however not optimal for repeated heating when devices for technically demanding applications of bioactive glass are manufactured (for example fibers, sintered fiber fabrics etc.).

The publication by Itala et al., published in Journal of Biomedical Materials Research (2001) 56 (2), pages 282-288, discloses a bioactive glass having the following composition:
SiO₂ in an amount of 53 wt-% of the starting oxides,
Na₂O in an amount of 6 wt-% of the starting oxides,
CaO in an amount of 22 wt-% of the starting oxides,
K₂O in an amount of 11 wt-% of the starting oxides,
MgO in an amount of 5wt-% of the starting oxides,
P₂O₅ in an amount of 2 wt-% of the starting oxides, and
B₂O₃ in an amount of 1 wt-% of the starting oxides.

This document does however not discuss the properties of the glass composition when heated repeatedly.

### OBJECTS AND SUMMARY OF THE INVENTION

The object of this invention is to provide a bioactive glass composition that can be repeatedly heat-treated without the crystallizing of the glass and losing its bioactive properties. A further object of this invention is to provide a bioactive glass composition that is suitable for manufacturing devices for technically demanding applications of bioactive glass.

### DETAILED DESCRIPTION OF THE INVENTION

The invention is disclosed in the appended claims.

The bioactive glass composition according to the invention is characterized in that the amount of
SiO₂ is 51-56 wt-% of the starting oxides,
Na₂O is 7-9 wt-% of the starting oxides,
CaO is 21-23 wt-% of the starting oxides,
K₂O is 10-12 wt-% of the starting oxides,
MgO is 1-4 wt-% of the starting oxides,
P₂O₅ is 0,5-1,5 wt-% of the starting oxides, and
B₂O₃ is 0-1 wt-% of the starting oxides,
provided that the total amount of Na₂O and K₂O is 17-20 wt-% of the starting oxides.

Thus, the invention concerns a bioactive glass composition that can be heat-treated even repeatedly.

The Applicants have indeed found that a bioactive glass having the above-mentioned composition has unexpected and surprisingly good heat-treating properties. The present invention is thus a selection invention of the above-mentioned invention disclosed in WO 96/21628. Indeed, the selected sub-range is narrow compared to the range disclosed in WO 96/21628, it is far removed from the end-points of said range of WO 96/21628 and it is a purposive selection since having an unexpected technical effect.

The amount of different oxides is given as weight percent of the starting oxides because some elements, such as sodium, evaporate during the heating. The amounts of the final oxides are however close to those of the starting oxides and in any case, the difference between the starting amounts and the final amounts is less than 5 percentage units, preferably less than 3 percentage units.

It is obvious to a person skilled in the art that the amounts of the oxides can be freely chosen within the above-mentioned limits. Indeed, the amount of SiO₂ can be for example 51,5, 52, 53,5, 55 or 56 wt-% of the starting oxides, the amount of Na₂O can be for example 7, 7,3, 7,7, 8, 8,5 or 9 wt-% of the starting oxides, the amount of CaO can be for example 21, 21,4, 21,7, 22, 22,6 or 23 wt-% of the starting oxides, the amount of K₂O can be for example 10, 10,5, 10,6, 11, 11,3, 11,7 or 12 wt-% of the starting oxides, the amount of MgO can be for example 1, 1,3, 1,9, 2,4, 2,7, 3,5 or 4 wt-% of the starting oxides, the amount of P₂O₅ can be for example 0,5, 0,7, 1, 1,2 or 1,5 wt-% of the starting oxides, and the amount of B₂O₃ can be for example 0, 0,4, 0,6, 0,9 or 1 wt-% of the starting oxides.

According to an embodiment of the invention, the amount of SiO₂ is 54-56 wt-% of the starting oxides

According to another embodiment of the invention, the inventive glass composition further comprises Al₂O₃ up to 1 wt-% of the starting oxides provided that the total amount of B₂O₃ and Al₂O₃ is 0,5-2,5 wt-% of the starting oxides.

According to yet another embodiment of the invention, the decrease in the amount of Na₂O and/or K₂O is compensated by the increase of the amount of Al₂O₃ and/or B₂O₃.

It is believed that the role of bioactive glass in bone formation is two-fold: to supply Ca²⁺ ions and to form a silica gel layer on the surface of the glass. This gel acts as a diffusion barrier, thus slowing down the leaching of ions from the glass and accordingly slowing down the formation of the new reaction layers and new body tissue. The silica gel is also acidic and may therefore irritate the tissues.

A further advantage of the bioactive glass composition according to the invention is that the primary reaction of a device manufactured from the inventive glass composition with the body tissue is "gentle", i.e. not so aggressive as with some traditional bioactive glasses. Indeed, firstly calcium phosphate is formed in the relatively thin layer into the silica gel on the surface of the glass, typically in about 6 hours and secondly, a calcium phosphate layer is formed on the layer of silica gel, typically in about 48-72 hours. During the formation of the primary calcium phosphate, the layer of silica gel on the surface of the inventive glass composition is essentially thinner than the corresponding layer on a traditional bioactive glass, for example as the one identified above.

In other words the bioactive glass composition according to the invention reacts in to an appropriate extent and does not dissolve too much, which is obviously an advantage in situations *in vivo.* On the other hand, a device manufactured from the inventive composition remains bioactive for a long period of time.

This advantage of the composition allows its use in target organs that are very sensible, such as cornea. The inventive glass composition reacts with the tissue in a gentle way, thereby reducing the chemical irritation due to the formation of the silica gel layer, for example. The properties of the inventive glass composition also allow it to be used in a powder having smaller particles than the traditional compositions, thus further decreasing the irritability of the composition.

Other suitable target organs are for example organs having a poor blood circulation such as sinuses or the bones of elderly patients. The present bioactive glass composition may also advantageously be used to recreate tissues that have disappeared due to an infection.

The bioactive glass composition according to the invention thus has an increased ability to react in a controlled and desired manner. Furthermore, it can be manufactured into any desired device according to conventional manufacturing methods and thus it may be used in applications requiring especially accurate devices and conditions.

Indeed, the bioactive glass having a composition according to the invention may be processed with any conventional methods. It may for example be firstly made into a solid glass that is further crushed. The composition according to the invention has the further advantage that it is possible to make granules thereof having a particularly well-controlled particle size distribution. These granules can be further heated to obtain spheres that may yet further be sintered to obtain a porous device of any desired shape. It is yet further possible to use the spheres or other particles of the bioactive glass for different casting processes such as pressure casting or for the casting of a thin sheet of glass with a process similar to the production of window glass.

A particularly preferred method for the treatment of the present bioactive glass composition is heating with laser since it allows localized yet high temperatures to be used in the melting of the glass.

The devices according to the invention may be in various forms, e.g., in the form of a particle, a disc, a film, a membrane, a tube, a hollow particle, a coating, a sphere, a semi sphere or a monolith, and they may have various applications.

Also fibres, granulates, woven and nonwoven mats, tissue-guiding devices as well as films may be manufactured. By tissue-guiding device a device is meant that has such properties that once in place in the patient's body it guides the formation of different types of tissues on different portions of the device. It may also be a device of a desired shape having various channels through its body in order to guide the formation of a vein in these locations.

Especially interesting forms of the present bioactive glass composition are fibre rovings, a perforated plate or sheet and a woven tissue having a precise profile. A perforated plate or sheet may be manufacture by casting or weaving and the diameter of the perforations is typically in the range of 10-500 µm. By a woven tissue having a precise profile it is meant a tissue wherein the position of the fibres is determined with a precision of micrometers.

The bioactive glass composition according to the invention may also be used for the coating of a device. The coating may be performed either by casting or dipping or a device may be coated with crushed particles of bioactive glass that is then sintered. The bioactive glass composition according to the invention may especially advantageously be used in the coating of ceramic materials since the heat expansion coefficients of the glass and ceramics do not significantly deviate from each other. It is also possible to use the present bioactive glass composition for the coating of metal such as titanium. A further advantage of the present composition is that it undergoes the treatment without crystallizing.

Tooth-implants, hip-implants, knee-implants, mini plates, external fixation pins, stents (e.g. for use in repair of blood vessels) or any other implants can be coated with the inventive glass composition.

The glass according to the present invention is advantageously prepared in atmospheric pressure and at temperatures of about 1360 °C. The healing time for making the glass melt is typically three hours. No protection gas is needed. When preparing the glass composition according to the present invention, the constituents are first melted together and then cooled down. The resulting solid material is then crushed and remelted in order to obtain a homogeneous material.

A porous device may also be manufactured by injecting pressurized gas into the glass melt, for example during the casting of the glass. If pressurized air is used, the conventional glass crystallizes due to the low temperature of the air. This problem does however not occur with the inventive glass composition and therefore both open- and closed-celled structures may be formed. The pores may further be filled by some active agents. Porosity of the bioactive glass does not only noticeably increase the total reacting surface of the glass but also allows a three-dimensional formation of the healing bone tissue.

The inventive glass composition can further be used in the manufacturing of different composites and devices consisting of at least two materials, such as a combination of bioactive glass and a metal or a ceramic material.

The bioactive glass composite may comprise different materials such as polymers, metals or ceramics. In applications in which the device needs to dissolve, it is preferable to use for example biopolymers. Either polymers based on renewable raw materials, e.g. cellulose, or synthetic polymers that are biodegradable, e.g. polylactides are meant by "biopolymer".

A composite may be formed using the inventive bioactive glass composition as a matrix and a ceramic material as reinforcing component. The inventive composition is especially suitable for matrix due to its crystallization properties. The inventive composition also glues the reinforcing particles or fibers strongly together. An implant manufactured from such a composite quickly becomes porous once in contact with the body tissue, a property that is desired in some applications, such as devices for tissue engineering.

The additives or reinforcements used in the composites may be in various forms such as fibres, woven or nonwoven mats, particles or hollow particles. They may also be porous or dense materials, and it is obvious that they are preferably biocompatible.

An especially advantageous use of the present glass composition is in the form of fibres. Indeed, the present composition may be drawn to a fibre at higher temperatures than the known bioactive glass compositions. Typically, the manufacturing temperature may be even 100 °C higher than for the conventional bioactive glass compositions. Higher manufacturing temperatures lead to fibres having a smaller diameter since the viscosity of the glass melt decreases with increasing temperature. Also, the manufacturing temperature is critical for the resulting fibre product since it is close to the softening temperature of the glass, thus close to the crystallization temperature. A fibre manufactured from the present composition has then been heat-treated three times and it still has the described properties.

A further advantage of the present glass composition is its better stability during storage. Indeed, the glass composition may react with the humidity of air during storage. Therefore, a glass composition according to the present invention which has a homogeneous structure will react uniformly and the product after storage still has predictable properties.

It was stated above that the amounts of the final oxides is close to those of the starting oxides. As an example, when the theoretical composition of the final glass was:
SiO₂ 53 wt-%,
P₂O₅ 2 wt-%,
CaO 22 wt-%,
Na₂O 6 wt-%,
K₂O 11 wt-%,
MgO 5 wt-% and
B₂O₃ 1 wt-%,
then the amounts of the oxides in the final bioactive glass composition were, as analysed by EDX (Energy dispersive X-ray analysis):
SiO₂ 55,17 wt-%,
P₂O₅ 2.11 wt-%,
CaO 21.53 wt-%,
Na₂O 5.64 wt-%,
K₂O 9.46 wt-%,
MgO 5.09 wt-% and
B₂O₃ 1.00 wt-%.

The present invention further relates to a method for manufacturing a repeatedly heat-treatable bioactive glass composition according to the present invention, the method being characterized in that it comprises the steps of
a) heating a mixture of starting materials to a temperature of 1350-1450 °C for a period of essentially three hours,
b) allowing the obtained melt to cool down to ambient temperature for at least twelve hours,
c) crushing the obtained glass composition into pieces,
d) reheating the crushed glass composition to a temperature of 1350-1450 °C for a period of essentially three hours, and
e) molding the obtained bioactive glass composition into desired shape and allowing it to cool down to ambient temperature.

The method according to the present invention thus comprises two steps of melting or heating the composition in order to obtain a homogeneous mixture. The final bioactive glass composition can be cast or mold to any desired shape such as directly into the form of a sheet or a rod that can be further made into fibre or into a solid block that is used in the conventional way, i.e. crushed into pieces and reheated to be mold.

In this specification, except where the context requires otherwise, the words "comprise", "comprises" and "comprising" means "include", "includes" and "including", respectively. That is, when the invention is described or defined as comprising specified features, various embodiments of the same invention may also include additional features.

The invention is described below in greater detail by the following, nonlimiting drawings.

### BRIEF DESCRIPTION OF THE DRAWINGS

Figure 1 illustrates an example of a device for tissue engineering comprising the inventive glass composition.
Figure 2 illustrates a cross-section of a bioactive fabric comprising the inventive glass composition.
Figure 3a illustrates the reaction of a fibre made from conventional bioactive glass when in contact with a body fluid.
Figure 3b illustrates the reaction of a fibre made from the inventive bioactive glass when in contact with a body fluid.
Figure 4a shows a scanning electron microscope picture of a bioactive glass fiber according to the present invention at a magnification of x 100.
Figure 4b shows a scanning electron microscope picture of a bioactive glass fiber according to the present invention at a magnification of x500.
Figure 5a shows a scanning electron microscope picture of a bioactive glass fiber according to the present invention at a magnification of x100 and after immersion in Tris for 7 days.
Figure 5b shows a scanning electron microscope picture of a bioactive glass fiber according to the present invention at a magnification of x500 and after immersion in Tris for 7 days.

### DETAILED DESCRIPTION OF THE DRAWINGS

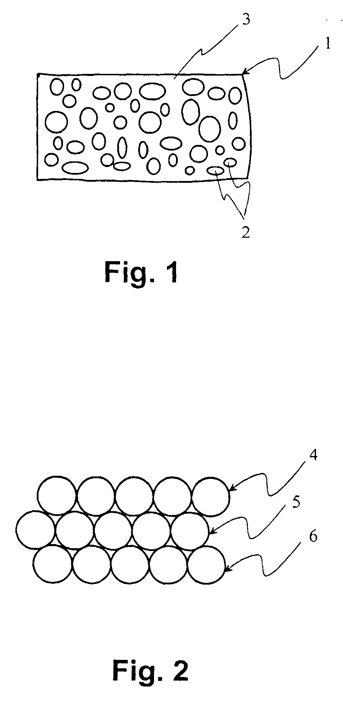

Figure 1 illustrates an example of a device for tissue engineering comprising the inventive glass composition. The device 1 comprises glass particles or short fibers 2 manufactured from the glass composition according to the present inventive bioactive glass composition and a matrix formed of a biopolymer 3. The degradation rate of the biopolymer is preferably superior to the dissolution rate of the bioactive glass. Therefore, the biopolymer 3 degrades and allows the formation of body tissues such as blood vessels whereas the bioactive glass remains essentially of a constant shape and size. This kind of tissue engineering device allows the formation of new tissues at the desired rate and shape while maintaining unchanged the cavity wherein the device is implanted. The biopolymer may also comprise a biological molecule such as growth hormone.

Figure 2 illustrates a cross-section of a bioactive fabric comprising the inventive glass composition. The fabric consists, in this embodiment, of three layers of fibers and can be either woven or nonwoven. The respective layers 4, 5 and 6 are manufactured from at least two different compositions of bioactive glass, each composition having a different bioactivity. The layer 4 may be manufactured from the inventive glass composition that maintains its bioactivity unchanged through the manufacturing of the fabric. The layers 5 and 6 may then be manufactured from glass compositions which bioactivities are either altered by the manufacturing process of the fabric or that are not bioactive at all.

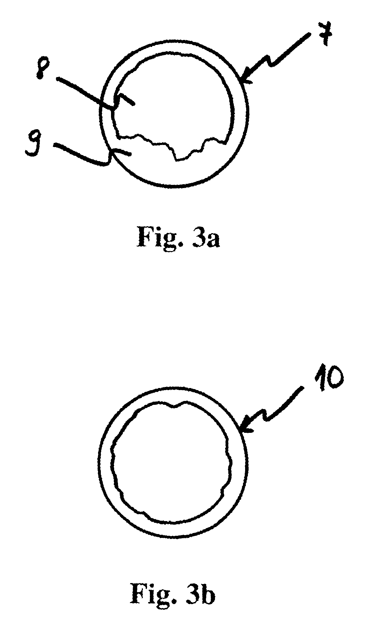

Figure 3a illustrates the reaction of a fiber 7 made from conventional bioactive glass when in contact with a body fluid. The Figure shows that the fiber has a heterogeneous structure consisting of a partially crystallized part 8 and an amorphous part 9. The amorphous part 9 has dissolved at a greater rate than the crystallized part 8 thus leading to an uneven cross-section of the reaction layers on the fiber.

Figure 3b illustrates the reaction of a fiber 10 made from the inventive bioactive glass when in contact with a body fluid. The homogeneous structure of the inventive material is clearly shown by the essentially even cross-section of the reaction layers on the fiber after reaction with a body fluid.

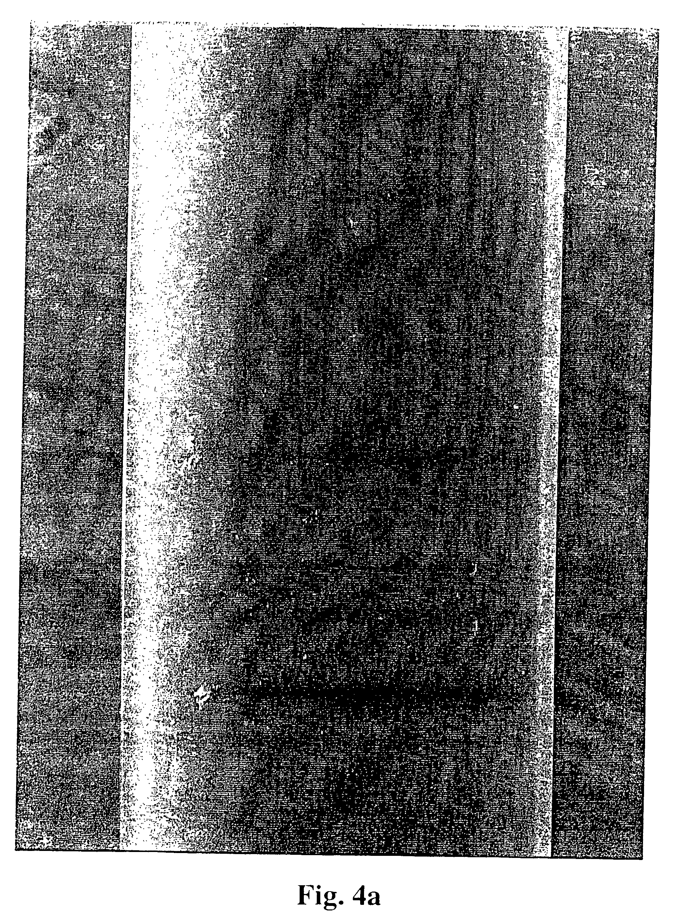

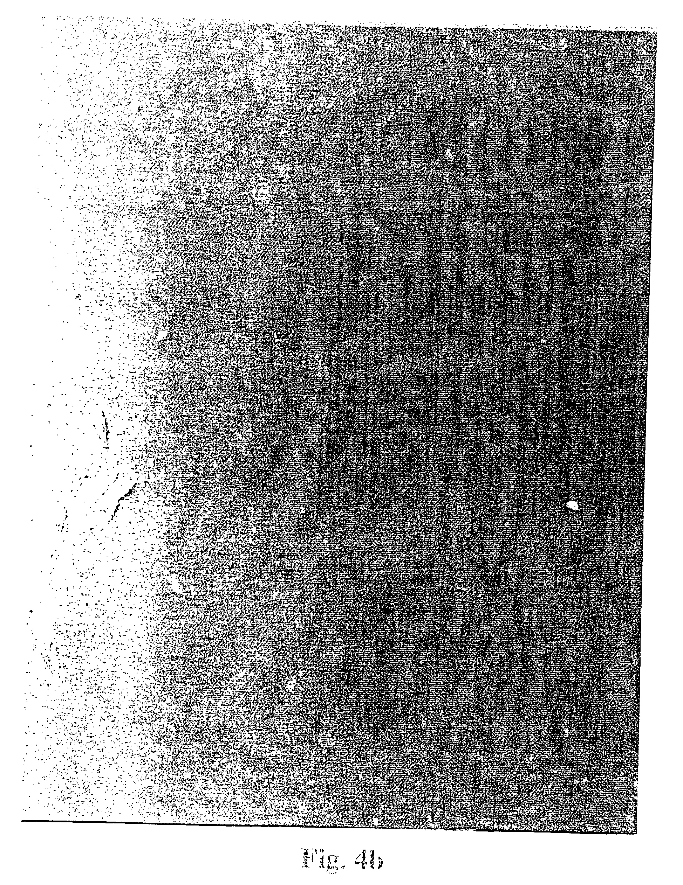

Figures 4a to 5b are discussed below.

### EXAMPLE

A composition consisting of:
165,00 g of SiO₂,
7,27 g of CaH(PO₄)x2H₂O,
108,21 g of CaCO₃,
41,04 g of Na₂CO₃,
48,42 g of K₂CO₃,
9,00 g of MgO, and
5,33 g of H₃BO₃
was heated to a temperature of 1360 °C and maintained in this temperature for a period of three hours. The melted composition wherein the carbonates had reacted forming oxides was allowed to cool down to ambient temperature overnight and the solid glass was crushed into pieces.

The crushed glass material was reheated to a temperature of 1360 °C and maintained in this temperature for a period of three hours. The resulting softened glass composition was cast into a mold and allowed to cool down to ambient temperature overnight. 300 g of bioactive glass according to the present invention was obtained. The composition of the glass was the following:
SiO₂ 55 wt-%,
P₂O₅ 1 wt-%,
CaO 21 wt-%,
Na₂O 8 wt-%,
K₂O 11 wt-%,
MgO 3 wt-% and
B₂O₃ 1 wt-%.

The bioactive glass composition obtained was used for drawing of a fiber by standard method and manufacturing a bioactive glass fabric by a nonwoven method. In said nonwoven method, the fibers were bonded to each other by using a thin layer of an aqucous solution of starch. Said solution also acted as a sizing agent thus increasing the strength of the fabric.

The resuming product was tested by immersing the fabric in Tris for 3, 5 and 7 days, respectively. Precipitation of calcium phosphate occurred at 5-7 days. Optical and X-ray analysis showed no crystals on or in the fibers.

Figures 4a to 5b illustrate the results of the testing. Figure 4a shows a scanning electron microscope (SEM) picture of a bioactive glass fiber according to the present invention at a magnification of x100 and Figure 4b shows the same sample at a magnification of x500, i.e. the clean surface for comparison.

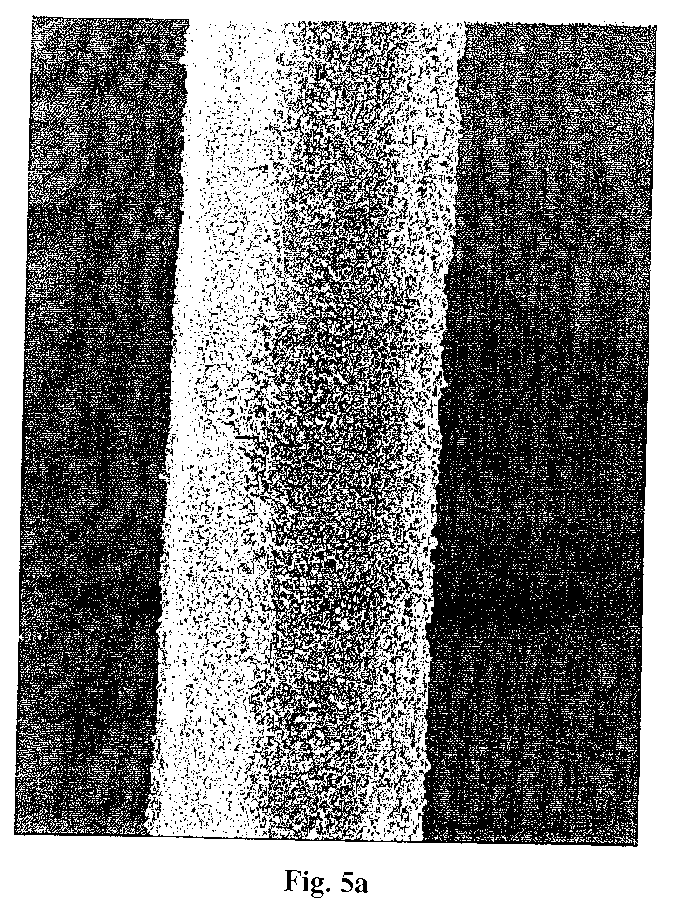

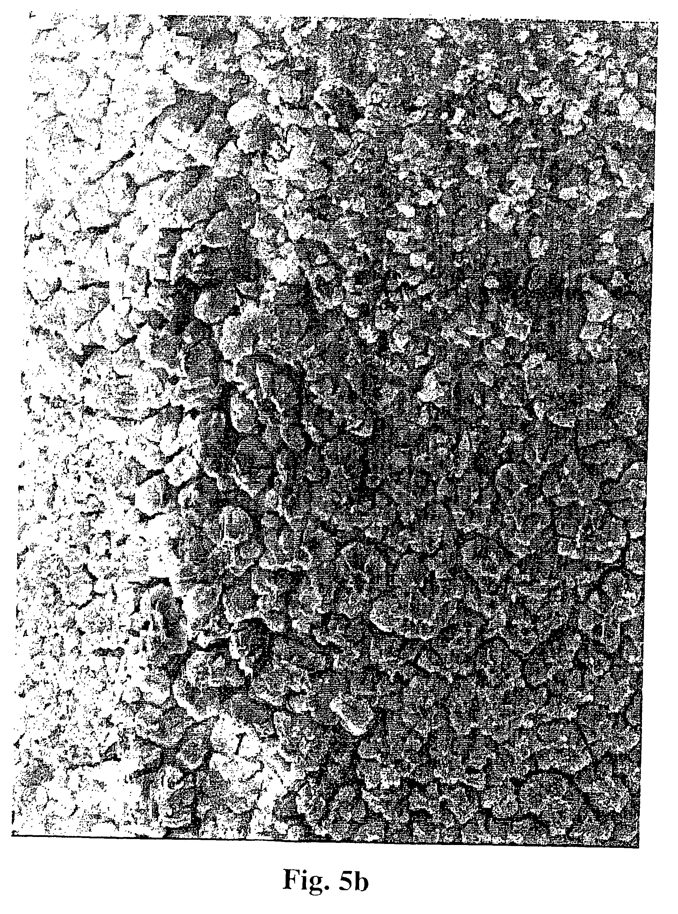

Figure 5a shows a scanning electron microscope picture of a bioactive glass fiber according to the present invention at a magnification of x100 and after immersion in Tris for 7 days and Figure 5b shows the same sample at a magnification of x500. In Figures 5a and 5b one can see a clear, irregular reaction surface that, in a mineral analysis, was identified as containing calsium phosphate (CaP) and silicon (Si). The bioactive glass according to the present invention thus reacts in a uniform manner, thus showing that the constitution of the glass is homogeneous.

## Claims

1. A bioactive glass composition comprising SiO₂, Na₂O, CaO, K₂O, MgO, P₂O₅ and B₂O₃, **characterized in that** the amount of
SiO₂ is 51-56 wt-% of the starting oxides,
Na₂O is 7-9 wt-% of the starting oxides,
CaO is 21-23 wt-% of the starting oxides,
K₂O is 10-12 wt-% of the starting oxides,
MgO is 1-4 wt-% of the starting oxides,
P₂O₅ is 0,5-1,5 wt-% of the starting oxides, and
B₂O₃ is 0-1 wt-% of the starting oxides,
provided that the total amount of Na₂O and K₂O is 17-20 wt-% of the starting oxides.

2. A bioactive glass composition according to claim 1, **characterized in that** the amount of SiO₂ is 54-56 wt-% of the starting oxides.

3. A bioactive glass composition according to claim 1 or 2, **characterized in that** it further comprises Al₂O₃ up to 1 wt-% of the starting oxides provided that the total amount of B₂O₃ and Al₂O₃ is 0,5-2,5 wt-% of the starting oxides.

4. A bioactive glass composition according to any of the preceding claims, **characterized in that** the decrease of the amount of Na₂O and/or K₂O is compensated by the increase of the amount of Al₂O₃ and/or B₂O₃.

5. Use of a bioactive glass composition according to any of the preceding claims in the coating of a device.

6. An implantable device, **characterized in that** it has been prepared from a bioactive glass composition according to any of the claims 1-4.

7. A fiber, **characterized in that** it has been prepared from a bioactive glass composition according to any of the claims 1-4

8. A sheet, **characterized in that** it has been prepared from a bioactive glass composition according to any of the claims 1-4.

9. A porous device, **characterized in that** it has been prepared from a bioactive glass composition according to any of the claims 1-4 by injecting pressurized gas into the molten glass composition.

10. A tissue engineering device, **characterized in that** it has been prepared from a bioactive glass composition according to any of the claims 1-4.

11. A method for manufacturing a repeatedly heat-treatable bioactive glass composition according to any of the claims 1-4, **characterized in that** it comprises the steps of
a) heating a mixture of starting materials to a temperature of 1350-1450 °C for a period of essentially three hours,
b) allowing the obtained melt to cool down to ambient temperature for at least twelve hours,
c) crushing the obtained solid glass into pieces,
d) reheating the crushed glass material to a temperature of 1350-1450 °C for a period of essentially three hours, and
e) molding the obtained bioactive glass composition into desired shape and allowing it to cool down to ambient temperature.

## Patentansprüche

1. Bioaktive Glaszusammensetzung, umfassend SiO₂, Na₂O, CaO, K₂O, MgO, P₂O₅ und B₂O₃, **dadurch gekennzeichnet , dass** die Menge an
SiO₂ 51-56 Gew.-% der Ausgangsoxide beträgt,
Na₂O 7-9 Gew.-% der Ausgangsoxide beträgt,
CaO 21-23 Gew.-% der Ausgangsoxide beträgt,
K₂O 10-12 Gew.-% der Ausgangsoxide beträgt,
MgO 1-4 Gew.-% der Ausgangsoxide beträgt,
P₂O₅ 0,5-1,5 Gew.-% der Ausgangsoxide beträgt und
B₂O₃ 0-1 Gew.-% der Ausgangsoxide beträgt,
unter der Maßgabe, dass die Gesamtmenge an Na₂O und K₂O 17-20 Gew.-% der Ausgangsoxide beträgt.

2. Bioaktive Glaszusammensetzung nach Anspruch 1, **dadurch gekennzeichnet, dass** die Menge an SiO₂ 54-56 Gew.-% der Ausgangsoxide beträgt.

3. Bioaktive Glaszusammensetzung nach Anspruch 1 oder 2, **dadurch gekennzeichnet, dass** sie des Weiteren Al₂O₃ in einer Menge bis zu 1 Gew.-% der Ausgangsoxide umfasst, unter der Maßgabe, dass die Gesamtmenge an B₂O₃ und Al₂O₃ 0,5-2,5 Gew.-% der Ausgangsoxide beträgt.

4. Bioaktive Glaszusammensetzung nach einem der voranstehenden Ansprüche, **dadurch gekennzeichnet, dass** die Abnahme der Menge an Na₂O und/oder K₂O durch die Zunahme der Menge an Al₂O₃ und/oder B₂O₃ kompensiert wird.

5. Verwendung einer bioaktiven Glaszusammensetzung nach einem der voranstehenden Ansprüche in der Beschichtung einer Vorrichtung.

6. Implantierbare Vorrichtung, **dadurch** gekennzeichn e t , dass sie aus einer bioaktiven Glaszusammensetzung nach einem der Ansprüche 1-4 hergestellt worden ist.

7. Faser, **dadurch gekennzeichnet, dass** sie aus einer bioaktiven Glaszusammensetzung nach einem der Ansprüche 1-4 hergestellt worden ist.

8. Platte, **dadurch gekennzeichnet, dass** sie aus einer bioaktiven Glaszusammensetzung nach einem der Ansprüche 1-4 hergestellt worden ist.

9. Poröse Vorrichtung, **dadurch gekennzeichnet, dass** sie aus einer bioaktiven Glaszusammensetzung nach einem der Ansprüche 1-4 durch Einpressen von Druckgas in die geschmolzene Glaszusammensetzung hergestellt worden ist.

10. Tissue Engineering-Vorrichtung, **dadurch gekennzeichnet, dass** sie aus einer bioaktiven Glaszusammensetzung nach einem der Ansprüche 1-4 hergestellt worden ist.

11. Verfahren zur Herstellung einer wiederholt wärmebehandelbaren bioaktiven Glaszusammensetzung nach einem der Ansprüche 1-4, **dadurch gekennzeichnet, dass** es die folgenden Stufen umfasst:
a) Erhitzen eines Gemisches aus Ausgangsmaterialien auf eine Temperatur von 1350-1450°C über einen Zeitraum von im Wesentlichen drei Stunden,
b) Abkühlenlassen der erhaltenen Schmelze auf Umgebungstemperatur über einen Zeitraum von mindestens zwölf Stunden,
c) Zerkleinern des erhaltenen festen Glases in Stücke,
d) erneutes Erwärmen des zerkleinerten Glasmaterials auf eine Temperatur von 1350-1450°C über einen Zeitraum von im Wesentlichen drei Stunden und
e) Formen der erhaltenen bioaktiven Glaszusammensetzung in die gewünschte Form und Abkühlenlassen auf Umgebungstemperatur.

## Revendications

1. Composition de verre bioactif comprenant SiO₂, Na₂O, CaO, K₂O, MgO, P₂O₅ et B₂O₃, **caractérisée en ce que** la quantité de :
- SiO₂ est de 51 à 56% en poids des oxydes de départ,
- Na₂O est de 7 à 9% en poids des oxydes de départ,
- CaO est de 21 à 23% en poids des oxydes de départ,
- K₂O est de 10 à 12% en poids des oxydes de départ,
- MgO est de 1 à 4% en poids des oxydes de départ,
- P₂O₅ est de 0,5 à 1,5% en poids des oxydes de départ,
- B₂O₃ est de 0 à 1% en poids des oxydes de départ, sous réserve que la quantité totale de Na₂O et de K₂O représente 17 à 20% en poids des oxydes de départ.

2. Composition de verre bioactif selon la revendication 1, **caractérisée en ce que** la quantité de SiO₂ est de 54 à 56% en poids des oxydes de départ.

3. Composition de verre bioactif selon la revendication 1 ou 2, **caractérisée en ce qu'**elle comprend en outre Al₂O₃ jusqu'à 1% en poids des oxydes de départ, sous réserve que la quantité totale de B₂O₃ et de Al₂O₃ représente de 0,5 à 2,5% en poids des oxydes de départ.

4. Composition de verre bioactif selon l'une quelconque des revendications précédentes, **caractérisée en ce que** la réduction de la quantité de Na₂O et/ou de K₂O est compensée par l'augmentation de la quantité de Al₂O₃ et/ou de B₂O₃.

5. Utilisation d'une composition de verre bioactif selon l'une quelconque des revendications précédentes dans le revêtement d'un dispositif.

6. Dispositif implantable, **caractérisé en ce qu'**il est préparé à partir d'une composition de verre bioactif selon l'une quelconque des revendications 1 à 4.

7. Fibre, **caractérisée en ce qu'**elle est préparée à partir d'une composition de verre bioactif selon l'une quelconque des revendications 1 à 4.

8. Feuille, **caractérisée en ce qu'**elle est préparée à partir d'une composition de verre bioactif selon l'une quelconque des revendications 1 à 4.

9. Dispositif poreux, **caractérisé en ce qu'**il est préparé à partir d'une composition de verre bioactif selon l'une quelconque des revendications 1 à 4 par injection de gaz sous pression dans la composition de verre fondu.

10. Dispositif d'ingénierie tissulaire, **caractérisé en ce qu'**il est préparé à partir d'une composition de verre bioactif selon l'une quelconque des revendications 1 à 4.

11. Procédé de fabrication d'une composition de verre bioactif selon l'une quelconque des revendications 1 à 4 pouvant être traitée à chaud de manière répétée, **caractérisé en ce qu'**il comprend les étapes consistant à :
a) chauffer un mélange de matières premières à une température de 1 350 à 1 450°C pendant essentiellement trois heures,
b) laisser refroidir la matière fondue ainsi obtenue à température ambiante pendant au moins douze heures,
c) écraser le verre solide ainsi obtenu pour le réduire en morceaux,
d) chauffer de nouveau la matière de verre écrasé à une température de 1 350 à 1 450°C pendant essentiellement trois heures, et
e) mouler la composition de verre bioactif ainsi obtenue à la forme souhaitée et la laisser refroidir à température ambiante.
